# EUROPEAN PATENT APPLICATION

(11) **EP 2 130 557 A1**
(43) Date of publication of application: **09.12.2009**
(21) Application number: 09007221.6
(22) Date of filing: 29.05.2009
(51) Int. Cl.: A61L 27/12, A61L 27/22, A61L 27/54

(54) **Bone Graft Substitute**

(30) Priority: 03.06.2008 JP 2008145820
(71) Applicant: GC Corporation, Itabashi-ku Tokyo 174-8585 (JP)
(72) Inventor: Yamamoto, Katsushi, Itabashi-ku Tokyo 174-8585 (JP); Yamanaka, Katsuyuki, Itabashi-ku Tokyo 174-8585 (JP); Sakai, Yuhiro, Itabashi-ku Tokyo 174-8585 (JP); Suda, Youko, Itabashi-ku Tokyo 174-8585 (JP); Kaneko, Tadashi, Itabashi-ku Tokyo 174-8585 (JP)
(74) Representative: Müller-Boré & Partner Patentanwälte

(57) **Abstract**

To provide a bone graft substitute having an appropriate absorption period in a living body and high osteoconductivity, the bone graft substitute contains a carbonate apatite and an osteoinductive factor, the osteo.inductive factor is preferably at least one kind selected from a group including BMP (a bone morphogenetic protein), GDF (a growth differentiation factor), TGF-β (a transformation growth factor), FGF (a fibroblast growth factor), IGF (an insulin-like growth factor), PDGF (a platelet-derived growth factor), BDNF (a brain-derived nerve growth factor), and NGF (a nerve growth factor), and the bone graft substitute has open pores, preferably having a diameter of 50 to 1000µm and/or a diameter of 0.001 to 5µm, with porosity of 20 to 80%.

## Description

The present invention relates to a bone graft substitute used for reinforcing or filling a defective part made after an affected part is extracted due to bone tumor or osteomyelitis, or a jawbone for embedding a dental implant.

A bone graft substitute is used for restoring a bone defect in orthopedics or a dental treatment field. As for the bone graft substitute, for example, Japanese Patent Application Laid-Open No. 5-237178 discloses a treatment in which a calcium phosphate-based material such as hydroxyapatite or β-tricalcium phosphate is used as an artificial material and filled in a defective part so as to induce bone regeneration.

The calcium phosphate-based material is a bioactive material, and is bonded directly with a bone so as to induce bone regeneration. However, in case of a large-scale bone defect for example, it is difficult to make sufficient restoration by only using an osteoconductivity of the bone graft substitute. In such the case, an autogeneous bone having higher osteoconductivity should be used. However, a collection amount of the autogeneous bone is limited, so that the application of the autogeneous bone is restricted. Further, since the autogeneous bone is collected from a healthy bone, there is a problem that an unnecessary burden is forced on a healthy part from which the autogeneous bone is collected. Therefore, for example, Japanese Patent Application Laid-Open No. 2001-137328 discloses a bone graft substitute developed by compounding a cell growth factor having an ability to induce bone formation and a calcium phosphate-based material as an artificial bone graft substitute.

A bone graft substitute compounded with hydroxyapatite and the growth factor has high osteoconductivity, and thus is excellent in bone formation around the graft substitute with respect to a large-scale bone defect. However, since the hydroxyapatite is not absorbed in a living body and remains as it is, there is a problem that a portion of hydroxyapatite, which is more fragile than a circumferential bone, might be broken when the hydroxyapatite is used in a load portion. Furthermore, the hydroxyapatite is non-absorbent, and thus might become an infection source after the elapse of years.

As for a bone graft substitute compounded with composing β-tricalcium phosphate and the growth factor, since the β-tricalcium phosphate has a lower osteoconductivity than that of hydroxyapatite, the growth factor just supplements the low osteoconductivity and the bone graft substitute is insufficient for restoring a large-scale bone defect. Furthermore, since the absorption mechanism of the β-tricalcium phosphate in a living body is operated by physical dissolving or foreign matter giant cells, absorbing of the β-tricalcium phosphate progresses quicker than bone formation in a case that bone formation ability is inferior due to an old age. Thus, there is a problem that a defective part is covered with an undesirable fiber connective tissue.

The present invention is directed to provide a bone graft substitute having an appropriate absorption period in a living body and high osteoconductivity.

The present inventors carried out earnest works to solve the aforementioned problems and, as a result, found out the followings to complete the present invention. When a material obtained by compounding a growth factor and a carbonic group-containing apatite having osteoconductivity equal to that of hydroxyapatite is used as a bone graft substitute, a bone is sufficiently regenerated even in case of a large-scale bone defect. Further, since the absorption mechanism of the carbonic group-containing apatite in a living body is operated by osteoclastic cells like a case of a remodeling of a living body bone, bone formation well-balanced with the absorption of the bone graft substitute can be done.

An aspect of the present invention is a bone graft substitute containing a carbonate apatite and an osteoinductive factor. The osteoinductive factor is preferably at least one kind selected from a group including BMP (a bone morphogenetic protein), GDF (a growth differentiation factor), TGF-β (a transformation growth factor), FGF (a fibroblast growth factor), IGF (an insulin-like growth factor), PDGF (a platelet-derived growth factor), BDNF (a brain-derived nerve growth factor), and NGF (a nerve growth factor). It is preferable that the bone graft substitute has open pores, the pores having either or both of a diameter of 50 to 1000µm and a diameter of 0.001 to 5µm, and has a porosity of 20 to 80%. Further, the content of a carbonic acid group is preferably 2 to 20% by weight.

The present invention is a bone graft substitute having an appropriate absorption period in a living body and high osteoconductivity.

A carbonate apatite used in the present invention is not restricted especially if it is safe to a living body and can maintain a shape in the living body for a fixed period of time. As for the size of a bone graft substitute used in the present invention, the bone graft substitute has preferably a granular shape having a diameter of 20µm or more or a block shape having one side of 20µm or more. If the size is less than 20µm, an inflammatory reaction may occur due to oligophagous cells.

The pores of the bone graft substitute according to the present invention are open pores, and classified to macro pores and micro pores, although they depend on a production method. The macro pores contribute to intrusion of cells and angiogenesis, and thus has a pore diameter of 50 to 1000µm, more preferably 50 to 500µm. If the pore diameter is less than 50µm, intrusion of cells and angiogenesis in the bone graft substitute may be difficult. If the pore diameter is more than 1000µm, strength of the bone graft substitute may decrease. Further, the micro pores have a pore diameter of 0.001 to 5µm, more preferably 0.01 to 2µm. If the pore diameter is less than 0.001µm, it is hard to have an osteoinductive factor thoroughly contained into fine spaces of the bone graft substitute. If the pore diameter is more than 5µm, it is hard to maintain the osteoinductive factor contained. In addition, the pore diameter can be measured by a scanning electron microscope picture or its image-processed image.

The porosity of the bone graft substitute is properly 20 to 80%. If the porosity is less than 20%, it is hard to have the osteoinductive factor thoroughly contained. If the porosity is more than 80%, the strength of the bone graft substitute tends to decrease. The porosity in the present invention is measured by a pore distribution measurement using a mercury intrusion method.

The content of a carbonic acid group in the carbonate apatite is properly 2 to 20% by weight of the whole apatite. If the content is less than 2% by weight, an appropriate absorption period is hardly obtained in a living body. On the other hand, it is hard to prepare a carbonate apatite having a proper size and containing more than 20% by weight of the carbonic acid group in a production process.

A production method of the carbonate apatite used in the present invention includes the steps of preparing a block shaped calcium material and a solution containing a phosphoric acid material, at least one of which has a carbonic acid group, and contacting the calcium material and the solution. Further, another method includes the steps of mixing a calcium material, a phosphoric acid material, and a carbonic acid material under a predetermined condition, producing a carbonate apatite powder, in which a part of a phosphoric acid group of hydroxyapatite is substituted with a carbonic acid group, by a so-called wet process, and burning it.

In the production method of the carbonate apatite through the steps of preparing a block shaped calcium material and a solution containing a phosphoric acid material, at least one of which has a carbonic acid group and contacting them, the block shaped calcium material is produced, and then dipped it in the solution containing a phosphoric acid material. At this time, since the carbonic acid group is contained in at least one of the block shaped calcium material and the solution containing a phosphoric acid material, the carbonate apatite having micro pores of 0.001 to 5µm is produced. Another method for preparing the block shaped calcium material includes the steps of dipping a sponge shaped material such as polyurethane foam in a calcium material slurry so as to adhere the calcium material to a sponge skeleton, and burning the urethane foam skeleton at a predetermined temperature so as to obtain a foam-shaped calcium material having open pores and a high porosity. By dipping the block shaped calcium material in the solution containing the phosphoric acid material, the carbonate apatite with macro pores having a pore diameter of 50 to 1000µm and micro pores having a pore diameter of 0.001 to 5µm can be produced.

In the production method including the steps of mixing a calcium material, a phosphoric acid material, and a carbonic acid material under a predetermined condition, producing a carbonate apatite powder, in which a part of a phosphoric acid group of hydroxyapatite is substituted with a carbonic acid group, by a so-called wet process, and burning it, for example, a carbonate apatite with micro pores having a pore diameter of 0.001 to 5µm can be produced by pressing and molding a carbonate apatite powder produced by the above method and sintering it.

The calcium material is a compound containing calcium. For example, the calcium material is calcium carbonate, tricalcium phosphate, tetracalcium phosphate, octacalcium phosphate, calcium nitrate, calcium hydrogen phosphate, calcium hydroxide, calcium oxide, calcium chloride, calcium silicate, a calcium halide such as calcium fluoride, an organic acid calcium salt such as calcium acetate, calcium hydride, or metal calcium.

The phosphoric acid material is a compound containing a phosphoric acid group. For example, the phosphoric acid material is disodium hydrogen phosphate, sodium dihydrogen phosphate, trisodium phosphate, diammonium hydrogen phosphate, ammonium dihydrogen phosphate, triammonium phosphate, dipotassium hydrogen phosphate, potassium dihydrogen phosphate, tripotassium phosphate, trimagnesium phosphate, an organic phosphoric acid such as dimethyl phosphate, a phosphoric acid metal salt such as copper phosphate, or phosphoric acid.

The carbonic acid material is a compound or material containing a carbonic acid group. For example, the carbonic acid material is calcium carbonate, ammonium carbonate, ammonium hydrogen carbonate, sodium carbonate, sodium hydrogen carbonate, potassium carbonate, potassium hydrogen carbonate, carbonated water, or carbon dioxide.

The bone graft substitute according to the present invention contains the carbonate apatite and the osteoinductive factor. The osteoinductive factor is preferably at least one kind selected from a group including BMP, GDF, TGF-β, FGF, IGF, PDGF, BDNF, and NGF, from the viewpoint of osteogenesis-inducing activity. The BMP is preferably BMP-2, 4, 5, 7, and 12 and FGF is preferably bFGF because these have high osteogenesis-inducing activity.

The amount of the osteoinductive factor in the bone graft substitute is generally 1 µg/g to 100 mg/g with respect to the bone graft substitute, although it depends on a kind of the factor used.

A method for containing the carbonate apatite and the osteoinductive factor is not restricted especially if it can uniformly disperse them without losing an activity of the osteoinductive factor. For example, a proper bone graft substitute can be easily obtained by adding a phosphate buffer solution, in which the osteoinductive factor is contained and suspended, to the carbonate apatite by dipping, impregnating, spraying, or dropping, and then drying (preferably, vacuum-drying or freeze-drying) the mixture. Preferably, the phosphate buffer solution is impregnated under reduced pressure, and then freeze-dried. At a time of compounding the osteoinductive factor, if a gelling material is added and compounded, holding ability of the osteoinductive factor increases so that it is preferable. The gelling material is prepared by dissolving 0.1 to 10% by weight of atelocollagen, hyaluronic acid, fibrin paste, carboxymethylcellulose, or gelatin in the phosphate acid buffer solution. These materials can be decomposed and absorbed in a living body. The amount of the gelling material is 0.5 ml/g to 3 ml/g.

### [Example]

### <Production of a bone graft substitute 1>

A calcium carbonate block having a diameter of 30 mm and a height of about 10 mm was obtained by uniaxially pressing and molding a calcium hydroxide powder of 9 g by using a circular metal mold having a diameter of 30 mm at an axial pressure of 20 kg/cm² so as to make a compact, and carbonizing the compact in a carbon dioxide gas flow at a relative humidity of 100%. Then, a carbonate apatite was obtained by pulverizing the calcium carbonate block to be a granular state having a diameter of 500 to 300µm, dipping the pulverized calcium carbonate granules in a disodium hydrogenphosphate solution having a concentration of 1 mol at 100°C for two weeks, and washing and drying it. The obtained carbonate apatite had a granular state having a diameter of 500 to 300µm, a carbonic acid group content of about 12% by weight, a pore diameter of about 0.1µm, and a porosity of 32%. Then, a bone graft substitute was obtained by dipping 1g of the carbonate apatite in the phosphate acid buffer solution in which 500µg of rh-BMP-2 was dissolved, taking out from the solution, and freeze-drying it. In addition, when the weight of the carbonate apatite was measured before and after freeze-drying it, 400µg of rh-BMP-2 was contained in the carbonate apatite.

### <Production of a bone graft substitute 2>

A polyurethane foam having a skeleton adhering α-type tricalcium phosphate was produced by preparing a suspension in which α-type tricalcium phosphate and distilled water were mixed at a weight ratio of 1:1, dipping a cubical polyurethane foam having one side of 10 mm in the suspension, and drying it. Then, the sintered foam-shaped α-type tricalcium phosphate was made by burning the polyurethane foam at 1500°C for 15 hours so as to remove the polyurethane form. Then, the carbonate apatite was obtained by dipping the foam-shaped α-type tricalcium phosphate in an aqueous solution in which sodium carbonate and disodium hydrogenphosphate are suspended, subjecting it to a hydrothermal treatment at 200°C for 48 hours, and washing and drying it. The obtained carbonate apatite had a cubical foam shape having one side of 10 mm, a carbonate content of about 6% by weight, open macro pores having a pore diameter of about 400µm, open micro pores having a pore diameter of about 0.3µm, and a porosity of about 75%. Then, a bone graft substitute was obtained by 1g of the carbonate apatite being impregnated with 1 ml of a gelling material in which carboxymethylcellulose of 1% by weight and 500µg/ml of bFGF were dissolved in a pH 7.4 phosphoric acid buffer solution.

### <Production of a bone graft substitute 3>

A solution of 1 L, in which 3 mol sodium hydrogencarbonate was dissolved in a 0.6 mol sodium hydrogenphosphate aqueous solution, and a 1 mol calcium acetate aqueous solution of 1 L were simultaneously dropped into ultrapure water kept at a temperature of 80°C at a dropping rate of 500 ml/Hr. During this dropping, the pH in the ultrapure water was controlled within 9.0 to 9.5 by 1N sodium hydroxide solution. After the dropping, the solution is kept for 12 hours at 80°C, and carbonate apatite powder was obtained by repeating filtrating of the solution and washing. The carbonate apatite powder was pulverized by a wet process using a zirconia pot for 24 hours so as to have an average particle diameter of about 0.5µm. 1g of the obtained carbonate apatite powder having an average particle diameter of about 0.5µm was filled in a metal mold for shaping so as to be pre-formed at 25 MPa, and then subjected to CIP molding at a CIP pressure of 600 MPa. Then, a carbonate apatite was obtained by increasing temperature to the obtained molded product at a rate of 5°C/min. and keeping it at 750°C for 2 hours so as to sinter it. The obtained carbonate apatite had a carbonic acid group content of about 7% by weight, open micro pores having a pore diameter of 0.5µm, and a porosity of about 21%. A bone graft substitute was obtained by mixing 1 g of the carbonate apatite and 1 ml of a gelling material in which atelocollagen of 2% by weight was dissolved in a pH 7.4 phosphoric acid buffer solution containing 500µg/ml of rh-BMP-7.

## Claims

1. A bone graft substitute containing a carbonate apatite and an osteoinductive factor.

2. The bone graft substitute as claimed in claim 1, wherein the osteoinductive factor is at least one kind selected from a group including BMP, GDF, TGF-β, FGF, IGF, PDGF, BDNF, and NGF.

3. The bone graft substitute as claimed in claim 1 or 2, wherein the bone graft substitute has open pores, the pores having either or both of a diameter of 50 to 1000µm and a diameter of 0.001 to 5µm, and a porosity is 20 to 80%.

4. The bone graft substitute as claimed in claim 1, 2 or 3, wherein the content of the carbonic acid group is 2 to 20% by weight.
